# EUROPEAN PATENT SPECIFICATION

(11) **EP 2 994 090 B1**
(45) Date of publication and mention of the grant of the patent: **06.12.2017**
(21) Application number: 13752667.9
(22) Date of filing: 08.05.2013
(51) Int. Cl.: A61K 8/24, A61K 8/73, A61Q 19/08

(54) **HYALURONIC ACID GELS CROSSLINKED BY SODIUM TRIMETAPHOSPHATE**
DURCH NATRIUMTRIMETAPHOSPHAT VERNETZTE HYALURONSÄUREGELE
GELS D'ACIDE HYALURONIQUE RÉTICULÉS PAR DU TRIMÉTAPHOSPHATE DE SODIUM

(43) Date of publication of application: 16.03.2016
(73) Proprietor: Allergan Industrie, 74370 Pringy (FR)
(72) Inventor: PIERRE, Sebastien, 74000 Annecy (FR)
(74) Representative: Hoffmann Eitle
(86) International application number: PCT/IB2013/001676
(87) International publication number: WO 2014/181147

(56) References cited:
- US-A1- 2003 148 995
- US-B1- 6 299 907
- DULONG V ET AL: "Hyaluronan-based hydrogels particles prepared by crosslinking with trisodium trimetaphosphate. Synthesis and characterization", CARBOHYDRATE POLYMERS, APPLIED SCIENCE PUBLISHERS, LTD. BARKING, GB, vol. 57, no. 1, 12 August 2004 (2004-08-12), pages 1-6, XP004522735, ISSN: 0144-8617, DOI: 10.1016/J.CARBPOL.2003.12.006
- DATABASE CA [Online] CHEMICAL ABSTRACTS SERVICE, COLUMBUS, OHIO, US; 2011, KIM, DONG-HWAN ET AL: "Preparation of hyaluronic acid microspheres with enhanced physical stability by double cross-link or alginate", XP002718533, retrieved from STN Database accession no. 2011:583527 & KIM, DONG-HWAN ET AL: "Preparation of hyaluronic acid microspheres with enhanced physical stability by double cross-link or alginate", YAKHAK HOECHI , 55(1), 69-74 CODEN: YAHOA3; ISSN: 0377-9556, 2011,

## Description

### BACKGROUND

The present invention generally relates to injectable compositions for aesthetic use, and more specifically relates to crosslinked hyaluronic acid-based dermal filler compositions.

Skin aging is a progressive phenomenon, occurs over time and can be affected by lifestyle factors, such as alcohol consumption, tobacco use and sun exposure. Aging of the facial skin can be characterized by atrophy, slackening, and fattening. Atrophy corresponds to a massive reduction of the thickness of skin tissue. Slackening of the subcutaneous tissues may lead to an excess of skin and ptosis can and lead to the appearance of drooping cheeks and eye lids. Fattening refers to an increase in excess weight by swelling of the bottom of the face and neck. These changes are typically associated with dryness, loss of elasticity, and rough texture.

Hyaluronan, also known as hyaluronic acid (HA) is distributed widely throughout the human body in connective and epithelial tissues and abundant in the different layers of the skin, where it has multiple functions such as, e.g., to ensure good hydration, to assist in the organization of the extracellular matrix, to act as a filler material, and to participate in tissue repair mechanisms. However, with age, the quantity of HA, collagen, elastin, and other matrix polymers present in the skin decreases. For example, repeated exposed to ultra violet light, e.g., from the sun, causes dermal cells to both decrease their production of HA as well as increase the rate of their degradation. This HA loss can result in various skin conditions such as, e.g., imperfects, defects, diseases and/or disorders, and the like. For instance, there is a strong correlation between the water content in the skin and levels of HA in the dermal tissue. As skin ages, the amount and quality of HA in the skin is reduced. These changes lead to drying and wrinkling of the skin.

Dermal fillers are useful in treating soft tissue conditions and in other skin therapies because the fillers can replace lost endogenous matrix polymers, or enhance/facilitate the function of existing matrix polymers, in order to treat these skin conditions. In the past, such compositions have been used in cosmetic applications to fill wrinkles, lines, folds, scars, and to enhance dermal tissue, such as, *e.g*., to plump thin lips, or fill-in sunken eyes or shallow cheeks. One common matrix polymer used in dermal filler compositions is HA. Because HA is natural to the human body, it is a generally well tolerated and a fairly low risk treatment for a wide variety of skin conditions. Unfortunately, some HA compositions are less stable to sterilization, such as heat sterilization, than may be desired.

CARBOHYDRATE POLYMERS, vol. 57, no. 1, 12 August 2004, pages 1-6 discloses hyaluronan based hydrogel particles prepared by crosslinking with sodium trimetaphosphate, which are suitable for use in the human body.

YAKHAK HOECHI, 2011, 55(1), pages 69-74 discloses hyaluronic acid microspheres suitable for cosmetic and biomedical use, which are crosslinked with trisodium trimetaphosphate.

US2003148995 discloses a method for crosslinking polysaccharides such as sodium hyaluronate to produce homogeneous hydrogels for use as dermal fillers.

US6299907 discloses the use of crosslinked starch in cosmetics and foodstuffs. The crosslinking agents may be sodium trimetaphosphate.

Current HA based dermal fillers are crosslinked with polyepoxides, such as 1,4-butanediol diglycidylether or polyvinylsulfones, such as divinylsulfone. These crosslinkers have some disadvantages.

The present invention provides new compositions comprising hyaluronic acid crosslinked with (tri)sodium trimetaphosphate.

### SUMMARY

The present invention provides compositions, for example, hydrogels, useful as injectable dermal fillers, comprising hyaluronic acid crosslinked with (tri)sodium trimetaphosphate (TMP).

In one aspect, the compositions are suitable for use as an injectable dermal filler. Further, the compositions are stable to heat sterilization, for example, autoclave sterilization.

Some embodiments include a method of making a composition useful as an injectable dermal filler, the method comprising the steps of crosslinking a hyaluronic acid with TMP.

### DETAILED DESCRIPTION

In accordance with a broad aspect of the invention, a crosslinker, such as, (tri)sodium trimetaphosphate (TMP), is added to an uncrosslinked hyaluronic acid to form a crosslinked hyaluronic acid-based composition useful as a dermal filler, for example, for wrinkle filling, volumizing the face, etc.

Hyaluronic acid is a non-sulfated glycosaminoglycan that enhances water retention and resists hydrostatic stresses. It is non-immunogenic and can be chemically modified in numerous fashions. Hyaluronic acid may be anionic at pH ranges around or above the pKa of its carboxylic acid groups. Unless clearly indicated otherwise, reference to hyaluronic acid, hyaluronan, or HA herein may include its fully protonated, or nonionic form as depicted below, as well as any anionic forms and salts of hyaluronic acid, such as sodium salts, potassium salts, lithium salts, magnesium salts, calcium salts, etc.

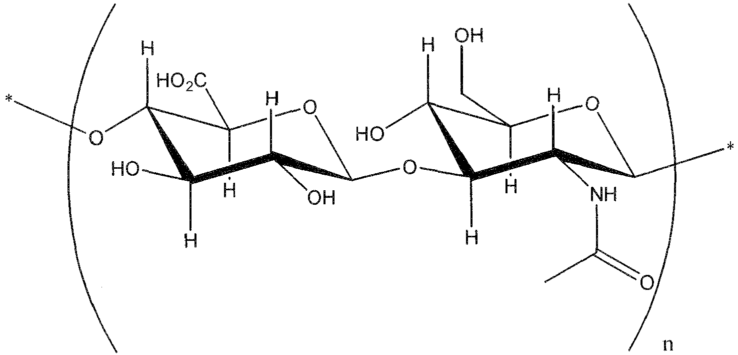

The HA useful in the invention may have any suitable molecular weight, such as an average molecular weight of about 5,000 Da to about 20,000,000 Da; about 300,000 Da to about 800,000 Da; or about 2,000,000 Da to about 5,000,000 Da.

In some embodiments, an HA comprises a mixture of high molecular weight HA, low molecular weight HA, and/or medium molecular weight HA, wherein the high molecular weight HA has a molecular weight greater than about 2,000,000 Da and wherein the low molecular weight HA has a molecular weight of less than about 1,000,000 Da, and the medium molecular weight HA has a molecular weight of between 1,000,000 Da and 2,000,000 Da.

In one embodiment, the HA in the compositions comprises at least 80% high molecular weight HA, for example, about 90% high molecular weight HA, for example, even 100% high molecular weight HA. In these embodiments, the high molecular weight HA may have a molecular weight of at least about 2.0 MDa, about 3.0 MDa, and up to about 3.5 MDa.

In some embodiments, an uncrosslinked HA fraction may optionally also be included in the compositions, for example, to improve the rheological properties of an HA composition to facilitate injection into skin. In aspects of this embodiment, a composition comprises an uncrosslinked HA where the uncrosslinked HA is present at a concentration of, e.g., about 2 mg/g, about 3 mg/g, about 4 mg/g, about 5 mg/g, about 6 mg/g, about 7 mg/g, about 8 mg/g, about 9 mg/g, about 10 mg/g, about 11 mg/g, about 12 mg/g, about 13 mg/g, about 13.5 mg/g, about 14 mg/g, about 15 mg/g, about 16 mg/g, about 17 mg/g, about 18 mg/g, about 19 mg/g, about 20 mg/g, about 40 mg/g, at least about 1 mg/g, at least about 2 mg/g, at least about 3 mg/g, at least about 4 mg/g, at least about 5 mg/g, at least about 10 mg/g, at least about 15 mg/g, at least about 20 mg/g, at least about 25 mg/g, at least about 35 mg/g, at most about 1 mg/g, at most about 2 mg/g, at most about 3 mg/g, at most about 4 mg/g, at most about 5 mg/g, at most about 10 mg/g, at most about 15 mg/g, at most about 20 mg/g, at most about 25 mg/g, about 1 mg/g to about 40 mg/g, about 7.5 mg/g to about 19.5 mg/g, about 8.8 mg/g to about 19 mg/g, about 9 mg/g to about 18 mg/g, about 10 mg/g to about 17 mg/g, about 11 mg/g to about 16 mg/g, or about 12 mg/g to about 15 mg/g. In some embodiments, the ratio of crosslinked HA to uncrosslinked HA is about 0.001 to about 100, about 0.005 to about 20, or about 0.01 to about 0.05.

In other embodiments, no uncrosslinked HA is present in the gels, or at least no uncrosslinked HA is added to the gels to improve rheology. For example, in some embodiments, the TMP-crosslinked HA is highly monophasic and extrudable without the need to add uncrosslinked HA in the final processing steps.

Others polysaccharides alternative to, or additional to, hyaluronic acid are contemplated and are considered to be within the scope of the invention. For example, the present invention may comprise a hydrogel composition comprising any suitable glycosaminoglycan polymer. The hydrogel composition disclosed herein can further comprise two or more different glycosaminoglycan polymers. As used herein, the term "glycosaminoglycan" is synonymous with "GAG" and "mucopolysaccharide" and refers to long unbranched polysaccharides consisting of a repeating disaccharide units. The repeating unit consists of a hexose (six-carbon sugar) or a hexuronic acid, linked to a hexosamine (six-carbon sugar containing nitrogen) and pharmaceutically acceptable salts thereof. Members of the GAG family vary in the type of hexosamine, hexose or hexuronic acid unit they contain, such as, *e.g.,* glucuronic acid, iduronic acid, galactose, galactosamine, glucosamine) and may also vary in the geometry of the glycosidic linkage. Any glycosaminoglycan polymer is useful in the hydrogel compositions disclosed herein with the proviso that the glycosaminoglycan polymer improves a condition of the skin when injected or when applied topically to the skin. Non-limiting examples of glycosaminoglycans include chondroitin sulfate, dermatan sulfate, keratan sulfate, hyaluronan. Non-limiting examples of an acceptable salt of a glycosaminoglycans includes sodium salts, potassium salts, magnesium salts, calcium salts, and combinations thereof.

In an embodiment, a hydrogel composition comprises a crosslinked glycosaminoglycan polymer where the crosslinked glycosaminoglycan polymer is present in an amount sufficient to improve a skin condition. In one aspect of this embodiment, a composition comprises a crosslinked chondroitin sulfate polymer, a crosslinked dermatan sulfate polymer, a crosslinked keratan sulfate polymer, a crosslinked heparan polymer, a crosslinked heparan sulfate polymer, or a crosslinked hyaluronan polymer. In other aspects of this embodiment, a composition comprises a crosslinked glycosaminoglycan where the crosslinked glycosaminoglycan represents, *e.g.,* about 1% by weight, about 2% by weight, about 3% by weight, about 4% by weight, about 5% by weight, about 6% by weight, about 7% by weight, about 8% by weight, or about 9%, or about 10% by weight, of the total glycosaminoglycan present in the composition. In yet other aspects of this embodiment, a composition comprises a crosslinked glycosaminoglycan where the crosslinked glycosaminoglycan represents, *e.g.,* at most 1% by weight, at most 2% by weight, at most 3% by weight, at most 4% by weight, at most 5% by weight, at most 6% by weight, at most 7% by weight, at most 8% by weight, at most 9% by weight, or at most 10% by weight, of the total glycosaminoglycan present in the composition. In still other aspects of this embodiment, a composition comprises a crosslinked glycosaminoglycan where the crosslinked glycosaminoglycan represents, *e.g.,* about 0% to about 20% by weight, about 1% to about 17% by weight, about 3% to about 15% by weight, or about 5% to about 10% by weight, for example, about 11% by weight, about 15% by weight or about 17% by weight, of the total glycosaminoglycan present in the composition.

In aspects of this embodiment, a hydrogel composition comprises a crosslinked glycosaminoglycan where the crosslinked glycosaminoglycan is present at a concentration of, *e.g.,* about 2 mg/g, about 3 mg/g, about 4 mg/g, about 5 mg/g, about 6 mg/g, about 7 mg/g, about 8 mg/g, about 9 mg/g, about 10 mg/g, about 11 mg/g, about 12 mg/g, about 13 mg/g, about 13.5 mg/g, about 14 mg/g, about 15 mg/g, about 16 mg/g, about 17 mg/g, about 18 mg/g, about 19 mg/g, or about 20 mg/g. In other aspects of this embodiment, a composition comprises a crosslinked glycosaminoglycan where the crosslinked glycosaminoglycan is present at a concentration of, *e.g.,* at least 1 mg/g, at least 2 mg/g, at least 3 mg/g, at least 4 mg/g, at least 5 mg/g, at least 10 mg/g, at least 15 mg/g, at least 20 mg/g, or at least 25 mg/g, or about 40 mg/g. In yet other aspects of this embodiment, a composition comprises a crosslinked glycosaminoglycan where the crosslinked glycosaminoglycan is present at a concentration of, *e.g.,* at most 1 mg/g, at most 2 mg/g, at most 3 mg/g, at most 4 mg/g, at most 5 mg/g, at most 10 mg/g, at most 15 mg/g, at most 20 mg/g, at most 25 mg/g, or at most 40 mg/g. In still other aspects of this embodiment, a composition comprises a crosslinked glycosaminoglycan where the crosslinked glycosaminoglycan is present at a concentration of, *e.g*., about 7.5 mg/g to about 19.5 mg/g, about 8.5 mg/g to about 18.5 mg/g, about 9.5 mg/g to about 17.5 mg/g, about 10.5 mg/g to about 16.5 mg/g, about 11.5 mg/g to about 15.5 mg/g, or about 12.5 mg/g to about 14.5 mg/g, up to about 40 mg/g.

For the crosslinker, the trimetaphosphate salt may comprise sodium trimetaphosphate, calcium trimetaphosphate, barium trimetaphosphate or a trivalent metal cation salt of trimetaphosphate.

The chemical structure of trimetaphosphate salt is shown below:

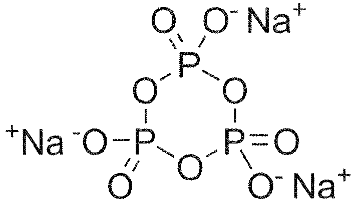

Crosslinking of TMP with a polysaccharide (ROH) in an alkaline medium is depicted below.

| | |
|---|---|
| **a** | ROH + NaOH → RONa |
| **b** | 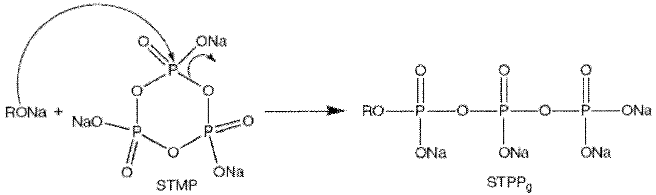 |
| **c** | 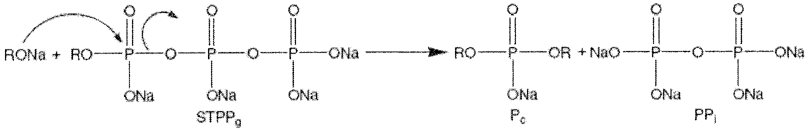 |
| **d** | 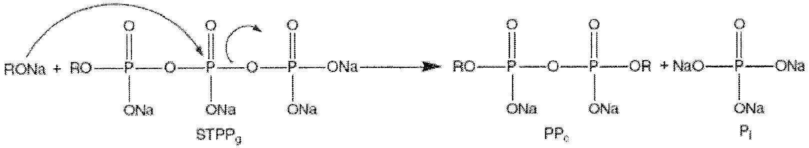 |

Alternatively, in some embodiments, the crosslinker may comprise metaphosphates other than trimetaphosphate, for example, tetrametaphosphates, pentametaphosphate and mostly hexametaphosphates.

An injectable, monophasic hydrogel formulation containing sodium hyaluronate (NaHA) crosslinked with a trimetaphosphate salt, is provided. Monophasic as used herein, refers to the composition being a single phase gel as opposed to a particulate gel. For example, a gel may be determined to be monophasic or not by use of a dye test where one volume of the gel to be tested is mixed with one volume of water, and then the mixture centrifuged. A droplet of red dye is deposed on top of the gel and diffusion of the dye through the swollen gel is observed during 10 minutes. If the dye does not penetrate the swollen gel over 10 minutes, the gel may generally be considered monophasic. If phase separation appears in the swollen gel, the dye will penetrate the liquid phase on top and diffuse downwards and the gel will generally be considered not monophasic, for example, biphasic.

In another aspect of the invention, an injectable, highly cohesive hydrogel formulation containing sodium hyaluronate (NaHA) crosslinked with a trimetaphosphate salt, is provided. Cohesive refers to the capacity of the gel to stay attached to itself, for example, meaning the resistance to cutting and the ability to elongate of the gel without breaking into pieces. This may be observed qualitatively by trying to cut the gel into pieces and by performing a tack test between the thumb and the index finger. In the tack test, gel is deposited on the thumb and the index finger. When slowly separating fingers, the distance that can be reached without the gel strand breaking between fingers is a measurement of cohesivity. The longer one can elongate the gel without breaking it, the more cohesive the gel is considered to be.

Hydrogel compositions in accordance with the invention may further and optionally comprise another agent or combination of agents that provide a beneficial effect when the composition is administered to an individual. Such beneficial agents include, without limitation, an antioxidant, an anti-itch agent, an anti-cellulite agent, an anti-scarring agent, an anti-inflammatory agent, an anesthetic agent, an anti-irritant agent, a vasoconstrictor, a vasodilator, an anti-hemorrhagic agent like a hemostatic agent or anti-fibrinolytic agent, a desquamating agent, a tensioning agent, an anti-acne agent, a pigmentation agent, an anti-pigmentation agent, or a moisturizing agent.

The hydrogel compositions disclosed herein may optionally comprise an anesthetic agent. An anesthetic agent is preferably a local anesthetic agent, *i.e.,* an anesthetic agent that causes a reversible local anesthesia and a loss of nociception, such as, *e.g.,* aminoamide local anesthetics and aminoester local anesthetics. The amount of an anesthetic agent included in a composition disclosed herein is an amount effective to mitigate pain experienced by an individual upon administration of the composition. As such, the amount of an anesthetic agent included in a composition disclosed in the present specification is between about 0.1% to about 5% by weight of the total composition. Non-limiting examples of anesthetic agents include lidocaine, ambucaine, amolanone, amylocaine, benoxinate, benzocaine, betoxycaine, biphenamine, bupivacaine, butacaine, butamben, butanilicaine, butethamine, butoxycaine, carticaine, chloroprocaine, cocaethylene, cocaine, cyclomethycaine, dibucaine, dimethysoquin, dimethocaine, diperodon, dycyclonine, ecgonidine, ecgonine, ethyl chloride, etidocaine, beta-eucaine, euprocin, fenalcomine, formocaine, hexylcaine, hydroxytetracaine, isobutyl p-aminobenzoate, leucinocaine mesylate, levoxadrol, lidocaine, mepivacaine, meprylcaine, metabutoxycaine, methyl chloride, myrtecaine, naepaine, octacaine, orthocaine, oxethazaine, parethoxycaine, phenacaine, phenol, piperocaine, piridocaine, polidocanol, pramoxine, prilocaine, procaine, propanocaine, proparacaine, propipocaine, propoxycaine, pseudococaine, pyrrocaine, ropivacaine, salicyl alcohol, tetracaine, tolycaine, trimecaine, zolamine, combinations thereof, and salts thereof. Non-limiting examples of aminoester local anesthetics include procaine, chloroprocaine, cocaine, cyclomethycaine, cimethocaine (larocaine), propoxycaine, procaine (novocaine), proparacaine, tetracaine (amethocaine). Non-limiting examples of aminoamide local anesthetics include articaine, bupivacaine, cinchocaine (dibucaine), etidocaine, levobupivacaine, lidocaine (lignocaine), mepivacaine, piperocaine, prilocaine, ropivacaine, and trimecaine. A composition disclosed herein may comprise a single anesthetic agent or a plurality of anesthetic agents. A non-limiting example of a combination local anesthetic is lidocaine/prilocaine (EMLA).

The compositions disclosed herein may comprise an anesthetic agent in an amount of, *e.g*., about 0.1%, about 0.2%, about 0.3%, about 0.4%, about 0.5%, about 0.6%, about 0.7%, about 0.8% about 0.9%, about 1.0%, about 2.0%, about 3.0%, about 4.0%, about 5.0%, about 6.0%, about 7.0%, about 8.0%, about 9.0%, or about 10% by weight of the total composition. In yet other aspects, a composition disclosed herein comprises an anesthetic agent in an amount of, *e.g.,* at least 0.1%, at least 0.2%, at least 0.3%, at least 0.4%, at least 0.5%, at least 0.6%, at least 0.7%, at least 0.8% at least 0.9%, at least 1.0%, at least 2.0%, at least 3.0%, at least 4.0%, at least 5.0%, at least 6.0%, at least 7.0%, at least 8.0%, at least 9.0%, or at least 10% by weight of the total composition. In still other aspects, a composition disclosed herein comprises an anesthetic agent in an amount of, *e.g.,* at most 0.1%, at most 0.2%, at most 0.3%, at most 0.4%, at most 0.5%, at most 0.6%, at most 0.7%, at most 0.8% at most 0.9%, at most 1.0%, at most 2.0%, at most 3.0%, at most 4.0%, at most 5.0%, at most 6.0%, at most 7.0%, at most 8.0%, at most 9.0%, or at most 10% by weight of the total composition. In further aspects, a composition disclosed herein comprises an anesthetic agent in an amount of, e.g., about 0.1% to about 0.5%, about 0.1% to about 1.0%, about 0.1% to about 2.0%, about 0.1% to about 3.0%, about 0.1% to about 4.0%, about 0.1% to about 5.0%, about 0.2% to about 0.9%, about 0.2% to about 1.0%, about 0.2% to about 2.0%, about 0.5% to about 1.0%, or about 0.5% to about 2.0% by weight of the total composition.

Hydrogel compositions disclosed herein may also optionally comprise an anti-oxidant agent. The amount of an anti-oxidant agent included in a composition disclosed herein is an amount effective to reduce or prevent degradation of a composition disclosed herein, such as, *e.g.,* enzymatic degradation and/or chemical degradation of the composition. As such, the amount of an anti-oxidant agent included in a composition may be between about 0.1% to about 10% by weight of the total composition. Non-limiting examples of antioxidant agents include a polyol, a flavonoid, a phytoalexin, an ascorbic acid agent, a tocopherol, a tocotrienol, a lipoic acid, a melatonin, a carotenoid, an analog or derivative thereof, and any combination thereof. A composition disclosed herein may comprise a single antioxidant agent or a plurality of antioxidant agents, a retinol, coenzyme, idebenone, allopurinol, gluthation, sodium selenite.

### EXAMPLE

### Preparation of a TMP-crosslinked HA gels

### Instruments:

Rheometer RS600 (G85). Oscillation method according to IP08020.

Imperial Loadcell 1000 from Mecmesin (G76) and Versa test column (G73).

Extrusion force method according to IP04175.

pH-meter (G90). pH method according to IP04172.

### Materials:

Trisodium trimetaphosphate and NaHA are readily available from commercial chemical suppliers.

### Conditions of preparation (parameter ranges):

HA concentration during crosslinking step : 50-250 mg/g.

In these specific examples, high molecular weight HA is 3-3.5 MDa, and low molecular weight HA is about 900 kDa are used as indicated in the Tables below.
% of Trisodium trimetaphosphate (for the crosslinking step): 50 to 300% compared to HA (weight of crosslinker versus weight of HA or NaHA).
Duration of crosslinking: 30min to 72h.
Temperature of crosslinking: 20°C to 70°C.

### Final specifications (ranges):

Final HA or NaHA concentration: 5-40 mg/g
final pH range: 6.0-8.0
Extrusion force : 5 - 15N with a 27G needle and a 0.8mL syringe at 12 to 50mm/min
Extrusion force : 5 - 20N with a 30G needle and a 0.8mL syringe at 12 to 50mm/min

### Preparation steps:

### (1) Hydration step

An aqueous solution made with TMP and NaOH 0.25M is contacted with, for example, poured onto the NaHA (for example, in the form of fibers or powder, mixes of different molecular weights) in order to obtain the defined NaHA concentration and % of TMP versus NaHA.

The NaHA is allowed to hydrate for 2h30 with regular mechanical homogenization. The pH may be adjusted to 11 by addition of NaOH 1N.

Alternatively, the NaHA fibers or powder are first hydrated in the minimum amount of NaOH 0.25M for 2h30 with mechanical homogenization. The TMP is then added to the a remaining volume of NaOH 0.25M, and this is blended with the NaHA gel for 30min by slow mechanical stirring.

### (2) Crosslinking Step

The mixture in step (1) is placed in a water bath at 50°C for 3H.

### (3) Neutralization step and homogenization step

A mixture of phosphate buffer and HCI is added to the crosslinked gel mixture to neutralize the solution at a pH around 7, to reach a NaHA concentration of about 25 mg/g, 38 mg/g, and 50 mg/g.

### (4) Dialysis step

The gel is then dialyzed against phosphate buffer for about 24H. The NaHA concentration is then adjusted to at least about 20 mg/g, for example, about 23 mg/g , about 24mg/g, about 25 mg/g, for example, up to about 30 mg/g.

### (5) Sterilization step

The gel stored in syringes is then sterilized with an autoclave.

**Table 1**

| Conditions of making various gels using process of Example 1 | | | | |
|---|---|---|---|---|
| Gel number | [NaHA]during crosslinking (mg/g) | HMW HA (%) | LMW HA (%) | Initial TMP content (wt% versus HA) |
| 3 | 90 | 100 | 0 | 100 |
| 4 | 90 | 100 | 0 | 200 |
| 6 | 90 | 80 | 20 | 300 |
| 7 | 90 | 100 | 0 | 300 |
| 10 | 100 | 80 | 20 | 250 |
| 11 | 90 | 100 | 0 | 150 |
| 12 | 90 | 100 | 0 | 200 |

**Table 2**

| Characteristics of the Gels made using process of Example 1 | | | | | | | | | |
|---|---|---|---|---|---|---|---|---|---|
| **Gel Number** | **Extrusion Force (N) 27G1/2, 0.8mL syr** | | **NaHA concentration (calculated value)** | **G' (Pa)** - **1Hz** | | **G" (Pa)** - **1Hz** | | **Tan Delta - 1Hz** | |
| | **Before ster.** | **After ster.** | | **Before ster.** | **After ster.** | **Before ster.** | **After ster.** | **Before ster.** | **After ster.** |
| **3** | 9.43±0.44 | 7.4 | ND | 220 | 144 | 122 | 110 | 0.56 | 0.77 |
| **4** | 8.88 | 8.85 | 23.8 | 547 | 336 | 226 | 209 | 0.41 | 0.62 |
| **6** | 15.4±1.9 | 13.9±0.67 | 23.2 | 197 | 112 | 72 | 47 | 0.37 | 0.42 |
| **7** | 11.9±2 | 6.6±0.6 | 22.3 | 433 | 242 | 144 | 150 | 0.33 | 0.62 |
| **10** | 9.5±0.7 | 7.9±0.6 | 22.7 | 344 | 185.5 | 132 | 118.5 | 0.390 | 0.639 |
| **11** | 10.7±0.4 | 9.7±0.2 | 24.0 | 104 | 50 | 90 | 62 | 0.867 | 1.219 |
| **12** | 11.3±0.5 | 9.5±0.1 | 24.0 | 246 | 141 | 121 | 93 | 0.49 | 0.65 |

A range of different crosslinked gels could be prepared using the methods of the present invention, the gels having a range of broad properties. It has been discovered that the gel elastic and viscous modulus increases with TMP content until TMP solubility prevents further crosslinking (generally, above 200 wt%) and yields fragmented gels with hard domains and non crosslinked domains.

A general characteristic of the present TMP-based gels is a very high viscous modulus but with good flowing properties.

While this invention has been described with respect to various specific examples and embodiments, it is to be understood that the invention is not limited thereto and that it can be variously practiced within the scope of the invention.

## Claims

1. A monophasic injectable dermal filler comprising:
a hyaluronic acid (HA) crosslinked with a trimetaphosphate (TMP).

2. The filler of claim 1 wherein the TMP comprises a salt of trimetaphosphate selected from the group consisting of sodium trimetaphosphate, calcium trimetaphosphate and barium trimetaphosphate.

3. The filler of claim 1 wherein the HA comprises a HA having a molecular weight of between about 2 MDa and about 3.5 MDa.

4. The filler of claim 1 wherein the HA comprises a HA having a molecular weight of at least about 3 MDa.

5. The filler of claim 1 wherein the concentration of TMP to HA in the filler is at least about 100 wt%.

6. The filler of claim 1 wherein the concentration of TMP to HA is between about 100 wt% and about 300 wt%.

7. The filler of claim 1 wherein the concentration of TMP to HA is between about 100 wt% and about 200 wt%.

8. The filler of claim 1 wherein the concentration of TMP to HA is about 150 wt%.

9. A method of making a monophasic injectable dermal filler comprising the steps of:
combining a hyaluronic acid (HA) with an aqueous solution containing a trimetaphosphate salt;
causing the HA to become crosslinked with the trimetaphosphate salt to form a gel;
homogenizing the gel; and
sterilizing the gel to obtain a monophasic injectable dermal filler.

10. The method of claim 9 wherein the salt of trimetaphosphate is selected from the group consisting of sodium trimetaphosphate, calcium trimetaphosphate and barium trimetaphosphate.

11. The method of claim 9 wherein the HA comprises a HA having a molecular weight of between about 2 MDa and about 3.5 MDa.

12. The method of claim 9 wherein the HA comprises a HA having a molecular weight of at least about 3 MDa.

13. The method of claim 9 wherein the concentration of TMP to HA in the filler is at least about 100 wt%.

14. The method of claim 9 wherein the concentration of TMP to HA in the filler is between about 100 wt% and about 300 wt%.

15. The method of claim 9 wherein the concentration of TMP to HA is between about 100 wt% and about 200 wt%, preferably 150 wt%.

## Patentansprüche

1. Monophasiger injizierbarer Dermalfiller, umfassend:
eine Hyaluronsäure (HA), vernetzt mit einem Trimetaphosphat (TMP).

2. Filler gemäß Anspruch 1, wobei das TMP ein Trimetaphosphatsalz umfasst, ausgewählt aus der Gruppe, bestehend aus Natriumtrimetaphosphat, Calciumtrimetaphosphat und Bariumtrimetaphosphat.

3. Filler gemäß Anspruch 1, wobei die HA eine HA mit einem Molekulargewicht von zwischen etwa 2 MDa und etwa 3,5 MDa umfasst.

4. Filler gemäß Anspruch 1, wobei die HA eine HA mit einem Molekulargewicht von wenigstens etwa 3 MDa umfasst.

5. Filler gemäß Anspruch 1, wobei die Konzentration von TMP zu HA in dem Filler wenigstens etwa 100 Gew.% beträgt.

6. Filler gemäß Anspruch 1, wobei die Konzentration von TMP zu HA zwischen etwa 100 Gew.% und etwa 300 Gew.% beträgt.

7. Filler gemäß Anspruch 1, wobei die Konzentration von TMP zu HA zwischen etwa 100 Gew.% und etwa 200 Gew.% beträgt.

8. Filler gemäß Anspruch 1, wobei die Konzentration von TMP zu HA etwa 150 Gew.% beträgt.

9. Verfahren zum Herstellen eines monophasigen injizierbaren Dermalfillers, umfassend die Schritte:
Kombinieren einer Hyaluronsäure (HA) mit einer wässrigen Lösung, enthaltend ein Trimetaphosphatsalz;
Veranlassen, dass die HA mit dem Trimetaphosphatsalz vernetzt wird, so dass ein Gel gebildet wird;
Homogenisieren des Gels; und
Sterilisieren des Gels, so dass ein monophasiger injizierbarer Dermalfiller erhalten wird.

10. Verfahren gemäß Anspruch 9, wobei das Trimetaphosphatsalz ausgewählt ist aus der Gruppe, bestehend aus Natriumtrimetaphosphat, Calciumtrimetaphosphat und Bariumtrimetaphosphat.

11. Verfahren gemäß Anspruch 9, wobei die HA eine HA mit einem Molekulargewicht von zwischen etwa 2 MDa und etwa 3,5 MDa umfasst.

12. Verfahren gemäß Anspruch 9, wobei die HA eine HA mit einem Molekulargewicht von wenigstens etwa 3 MDa umfasst.

13. Verfahren gemäß Anspruch 9, wobei die Konzentration von TMP zu HA in dem Filler wenigstens etwa 100 Gew.% beträgt.

14. Verfahren gemäß Anspruch 9, wobei die Konzentration von TMP zu HA in dem Filler zwischen etwa 100 Gew.% und etwa 300 Gew.% beträgt.

15. Verfahren gemäß Anspruch 9, wobei die Konzentration von TMP zu HA zwischen etwa 100 Gew.% und etwa 200 Gew.%, vorzugsweise 150 Gew.%, beträgt.

## Revendications

1. Produit de comblement dermique injectable monophasique comprenant :
un acide hyaluronique (AH) réticulé avec un trimétaphosphate (TMP).

2. Produit de comblement selon la revendication 1 dans lequel le TMP comprend un sel de trimétaphosphate sélectionné dans le groupe constitué de trimétaphosphate de sodium, de trimétaphosphate de calcium et de trimétaphosphate de baryum.

3. Produit de comblement selon la revendication 1, dans lequel l'AH comprend un AH ayant un poids moléculaire entre environ 2 MDa et environ 3,5 MDa.

4. Produit de comblement selon la revendication 1, dans lequel l'AH comprend un AH ayant un poids moléculaire d'au moins environ 3 MDa.

5. Produit de comblement selon la revendication 1, dans lequel la concentration de TMP rapportée à l'AH dans le produit de comblement est d'au moins environ 100 % en poids.

6. Produit de comblement selon la revendication 1, dans lequel la concentration de TMP rapportée à l'AH est entre environ 100 % en poids et environ 300 % en poids.

7. Produit de comblement selon la revendication 1, dans lequel la concentration de TMP rapportée à l'AH est entre environ 100 % en poids et environ 200 % en poids.

8. Produit de comblement selon la revendication 1, dans lequel la concentration de TMP rapportée à l'AH est d'environ 150 % en poids.

9. Procédé de fabrication d'un produit de comblement dermique injectable monophasique comprenant les étapes de :
combiner un acide hyaluronique (AH) avec une solution aqueuse contenant un sel de trimétaphosphate ;
amener l'AH à être réticulé avec le sel de trimétaphosphate pourformer un gel ;
homogénéiser le gel ; et
stériliser le gel pour obtenir un produit de comblement dermique injectable monophasique.

10. Procédé selon la revendication 9, dans lequel le sel de trimétaphosphate est sélectionné dans le groupe constitué de trimétaphosphate de sodium, de trimétaphosphate de calcium et de trimétaphosphate de baryum.

11. Procédé selon la revendication 9, dans lequel l'AH comprend un AH ayant un poids moléculaire entre environ 2 MDa et environ 3,5 MDa.

12. Procédé selon la revendication 9, dans lequel l'AH comprend un AH ayant un poids moléculaire d'environ 3 MDa.

13. Procédé selon la revendication 9, dans lequel la concentration de TMP rapportée à l'AH dans le produit de comblement est d'au moins environ 100 % en poids.

14. Procédé selon la revendication 9, dans lequel la concentration de TMP rapportée à l'AH dans le produit de comblement est entre environ 100 % en poids et environ 300 % en poids.

15. Procédé selon la revendication 9, dans lequel la concentration de TMP rapportée à l'AH est entre environ 100 % en poids et environ 200 % en poids, de préférence 150 % en poids.
